**Europäisches Patentamt**

**European Patent Office**

**Office européen des brevets**

(11) Veröffentlichungsnummer: **0 101 781**
**B1**

(19)

(12) # EUROPÄISCHE PATENTSCHRIFT

(45) Veröffentlichungstag der Patentschrift:
**20.11.86**

(51) Int. Cl.⁴: **A 61 B 17/02**

(21) Anmeldenummer: **83100735.6**

(22) Anmeldetag: **27.01.83**

(54) **Wundhaken für chirurgische Zwecke.**

(30) Priorität: **30.07.82 DE 8221649 U**

(43) Veröffentlichungstag der Anmeldung:
**07.03.84 Patentblatt 84/10**

(45) Bekanntmachung des Hinweises auf die Patenterteilung:
**20.11.86 Patentblatt 86/47**

(84) Benannte Vertragsstaaten:
**DE FR GB IT**

(56) Entgegenhaltungen:
**DE - A - 2 128 855**
**DE - A - 2 302 614**
**DE - A - 3 023 266**
**US - A - 2 235 979**
**US - A - 2 296 793**
**US - A - 4 226 228**

(73) Patentinhaber: **W.C. Heraeus GmbH,**
**Heraeusstrasse 12 - 14, D-6450 Hanau / Main (DE)**

(72) Erfinder: **Wendt, Dieter, Fritz-Schubert-Ring 1,**
**D-6454 Bruchköbel (DE)**
Erfinder: **Gerloff, Peter H., Dipl.-Ing., Im**
**Schwalbengrund 22, D-6463 Freigericht 1 (DE)**
Erfinder: **Koog, Willi, Im Ellenbügel 41,**
**D-6456 Langenselbold (DE)**

(74) Vertreter: **Heinen, Gerhard, Dr., Heraeusstrasse 12-14,**
**D-6450 Hanau/Main (DE)**

ACTORUM AG

## Beschreibung

Die Erfindung betrifft einen Wundhaken aus lichtleitendem Werkstoff für chirurgische Zwecke mit einem Hakenblatt, das eine einem Wundbereich zukehrbare Lichtaustrittsfläche aufweist, die derart strukturiert ist, dass wenigstens teilweise der Grenzwinkel der Totalreflexion unterschritten wird, und mit einem Handgriff, der mit dem Hakenblatt verbunden ist, und mit einem Kupplungsstück zur mechanischen und optischen Kupplung eines Faser- oder Flüssigkeits-Lichtleiters.

Ein derartiger Wundhaken ist aus der deutschen Offenlegungsschrift 3 023 266 bekannt.

Der vorliegenden Erfindung liegt die Aufgabe zugrunde, einen Wundhaken zu schaffen, der eine einfache, aber sichere, leicht handhabbare Verbindung mit dem Lichtleiter ermöglicht.

Erfindungsgemäss wird die Aufgabe für einen Wundhaken der eingangs charakterisierten Art dadurch gelöst, dass der Handgriff hohl ausgebildet ist und der so gebildete Hohlraum sowohl eine Führung für den Lichtleiter als auch eine dessen Lichtaustrittsende aufnehmende Kupplung aufweist. Zweckmässigerweise ist die Führung als ein in den Hohlraum des Handgriffes sich erstreckender Vorsprung ausgebildet, die sich vorteilhafterweise in Längsrichtung des Handgriffs erstreckt. Vorzugsweise weist der Wundhaken eine rohrförmige Kupplung zur Aufnahme des Lichtleiters auf. Das eine Ende der Kupplung ragt in das dem Hakenblatt zugekehrte Ende des Handgriffs hinein. Zweckmässigerweise weist der Handgriff im Bereich der Kupplung eine Öffnung auf. Der Handgriff weist bevorzugt im Bereich des dem Hakenblatt zugekehrten Endes wenigstens einen Fingerhaken und/oder an dem Hakenblatt abgekehrten Ende einen Wulst auf. Vorzugsweise ist das dem Hakenblatt zugekehrte Ende des Handgriffs verjüngt ausgebildet. Das Hakenblatt mit dem Handgriff ist vorteilhafterweise einstückig ausgebildet. Die Vorteile derartiger Wundhaken liegen darin, dass sie einstückig ausgebildet kostengünstig als Spritzteil mit einem Einlegeteil hergestellt werden können, dass sie durch die Ausbildung des Handgriffes gut handhabbar sind, dass die Lichtverluste durch die räumliche Anordnung der Kupplung gering sind, dass keinerlei Blendwirkungen für den Benutzer auftreten und dass durch die vorgesehene örtliche Lage des Lichtaustrittsende des Lichtleiters eine stets gleichbleibende Lichteinspeisung sichergestellt wird.

Ein Ausführungsbeispiel ist in den Zeichnungen dargestellt und nachfolgend beschrieben. Es zeigt:

Figur 1 einen Wundhaken in perspektivischer Ansicht
Figur 2 eine Draufsicht auf den Handgriff des Wundhakens
Figur 3 einen Längsschnitt durch den Handgriff und das Hakenblatt.

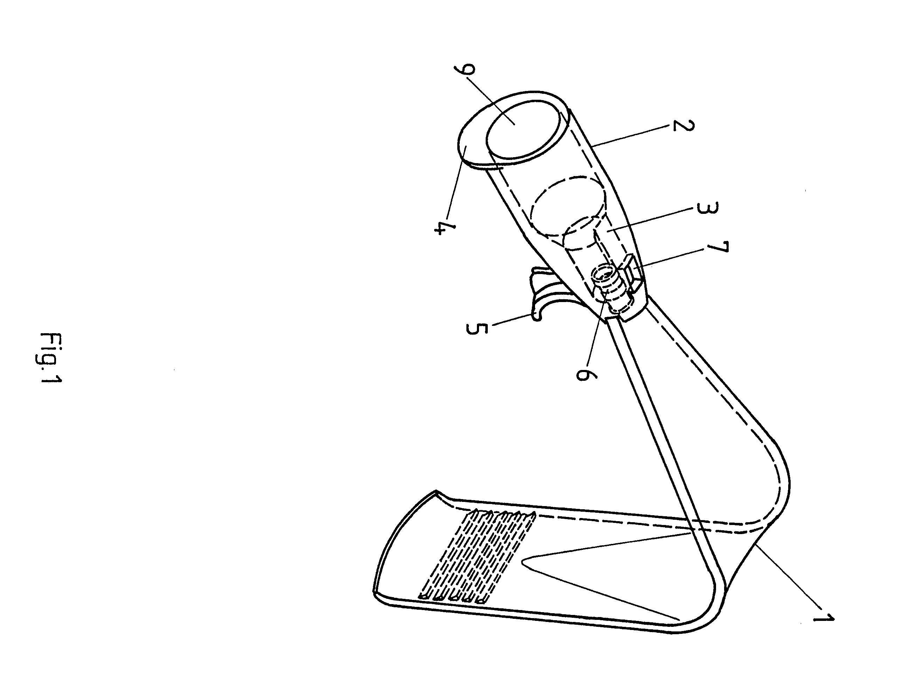

Figur 1 zeigt den Wundhaken in seiner Gesamtheit mit dem Hakenblatt 1 und dem Handgriff 2. Am Handgriff 2 ist an dem dem Hakenblatt abgekehrten Ende ein Wulst 4 angeordnet. Im Bereich der Kupplung 6 weist der Handgriff 2 auf der Oberseite eine fensterähnliche Öffnung 7 auf. Der Hohlraum 9 und die Führung 3 sind gestrichelt angedeutet.

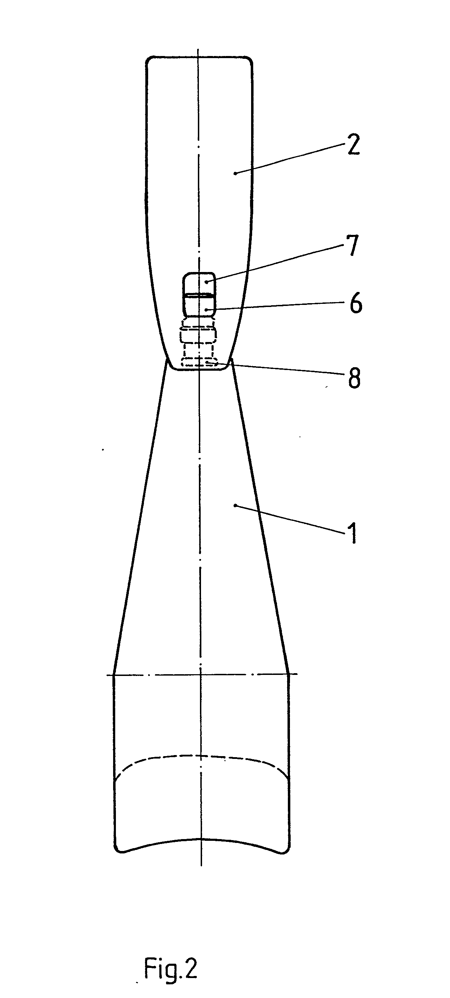

Der Handgriff 2 und ein Teil des Hakenblattes 1 sind in der Draufsicht der Figur 2 dargestellt. Sie zeigt die fensterähnliche Öffnung 7 und die dahinterliegende röhrenförmige Kupplung 6.

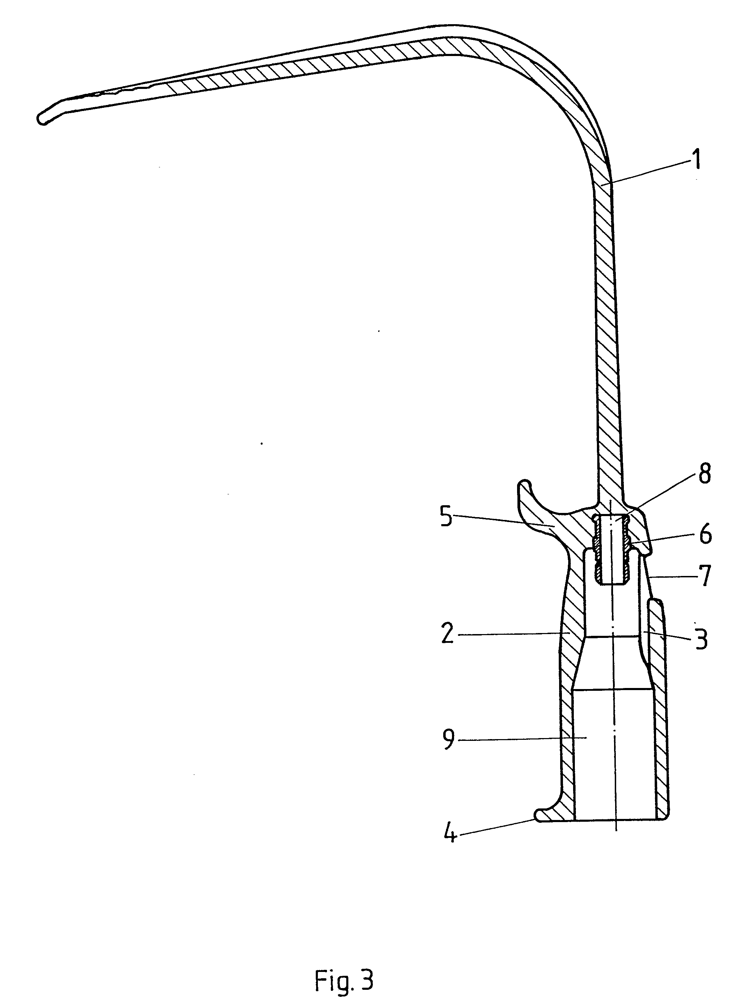

Die Figur 3 zeigt die räumliche Anordnung der Kupplung 6 und der im Hohlraum 9 des Handgriffs 2 angeordneten Führung 3, die sich in axialer Richtung erstreckt.

Der Wundhaken ist einstückig aus einem lichtleitenden Werkstoff mit einem Einlegeteil, der Kupplung 6, gespritzt. Die rohrförmige metallische Kupplung 6 weist eine lichtdurchlässige Scheibe auf der dem Lichtleiter zugewandten Seite auf. Durch den Fingerhaken 5, die Verjüngung und den Wulst 4, sowie die gesamte Anordnung Handgriff-Hakenblatt ist der Wundhaken gut zu handhaben. Die Dicke des Hakenblattes nimmt aus Festigkeits- und aus Schwerpunktsgründen zu dem Lichtaustrittsende hin ab. Die in dem hohlen Handgriff 2 angeordnete Führung 3 ermöglicht ein einfaches Einführen des Steckers des Lichtleiters. Durch die Öffnung 7 wird ein unbeabsichtigtes Lösen des Steckers von der Kupplung 6 erschwert, das gewollte Lösen jedoch ermöglicht. An der Ausstrahlungsöffnung 8 austretende Lichtstrahlen gelangen durch den unteren rechteckförmigen Querschnitt nicht sofort an die Oberfläche, so dass der Benutzer nicht geblendet wird und nur geringe Lichtverluste auftreten.

Das Hakenblatt und der Handgriff sind aus einem Material hergestellt. Es ist allerdings auch möglich für Hakenblatt und Handgriff unterschiedliche Materialien zu verwenden. Zur Korrektur der Farbtemperatur des Lichtes kann insbesondere das Material des Hakenblattes leicht eingefärbt werden.

Selbstverständlich kann die Form des Hakenblattes je nach anwendungstechnischen Erfordernissen hinsichtlich Länge, Breite und Krümmung gewählt werden. Die Lichtaustrittsfläche kann dann auf die Hakenform entsprechend abgestimmt werden.

## Patentansprüche

1. Wundhaken aus lichtleitendem Werkstoff für chirurgische Zwecke mit einem Hakenblatt, das eine einem Wundbereich zukehrbare Lichtaustrittsfläche aufweist, die derart strukturiert ist, das wenigstens teilweise der Grenzwinkel der Totalreflexion unterschritten wird, und mit einem Handgriff, der mit dem Hakenblatt verbunden ist, und mit einem Kupplungsstück zur mechanischen und optischen Kupplung eines Faser- oder Flüssigkeits-Lichtleiters, dadurch gekennzeichnet, dass der Handgriff (2) hohl ausgebildet ist und der so gebildete Hohlraum (9) sowohl eine Führung (3) für den Lichtleiter als auch eine dessen Lichtaustrittsende aufnehmende Kupplung (6) aufweist.

2. Wundhaken nach Anspruch 1, dadurch gekennzeichnet, dass die Führung (3) als ein sich in den Hohlraum (9) des Handgriffs (2) erstreckender Vorsprung ausgebildet ist.

3. Wundhaken nach Anspruch 1 oder 2, dadurch gekennzeichnet, dass sich die Führung (3) in Längsrichtung des Handgriffs (2) erstreckt.

4. Wundhaken nach einem der Ansprüche 1 bis 3, dadurch gekennzeichnet, dass die Kupplung (6) zur Aufnahme des Lichtaustrittsendes des Lichtleiters rohrförmig ausgebildet ist.

5. Wundhaken nach einem der Ansprüche 1 bis 4, dadurch gekennzeichnet, dass der Handgriff (2) im Bereich der Kupplung (6) eine Öffnung (7) aufweist.

6. Wundhaken nach einem der Ansprüche 1 bis 5, dadurch gekennzeichnet, dass der Handgriff (2) im Bereich des dem Hakenblatt (1) zugekehrten Endes wenigstens einen Fingerhaken (5) aufweist.

7. Wundhaken nach einem der Ansprüche 1 bis 6, dadurch gekennzeichnet, dass der Handgriff (2) an dem dem Hakenblatt (1) abgekehrten Ende einen Wulst (4) aufweist.

8. Wundhaken nach einem der Ansprüche 1 bis 7, dadurch gekennzeichnet, dass wenigstens das dem Hakenblatt (1) zugekehrte Ende des Handgriffes (2) verjüngt ausgebildet ist.

9. Wundhaken nach einem der Ansprüche 1 bis 8, dadurch gekennzeichnet, dass das Hakenblatt (1) mit dem Handgriff (2) einstückig ausgebildet ist.

## Claims

1. Retractor hook of light-conducting material for surgical purposes, comprising a hook blade which has a light emitting surface which may be directed towards a wound area, which is so structured as to pass at least partially below the limiting angle of total reflection, and a handle which is connected to the retractor blade, and a coupling member for mechanical and optical connection of a fibre or fluid light conductor characterised in that the handle (2) is of hollow construction and the cavity (9) so formed has a locator (3) for the light conductor as well as a connector (6) receiving its light emitting extremity.

2. Retractor hook according to claim 1, characterised in that the locator (3) is constructed as a projection extending into the cavity (9) of the handle (2).

3. Retractor hook according to claim 1 or 2, characterised in that the locator (3) extends in the longitudinal direction of the handle (2).

4. Retractor hook according to one of the claims 1 to 3, characterised in that the connector (6) for reception of the light emitting extermity of the light conductor is tubularly constructed.

5. Retractor hook according to one of the claims 1 to 4, characterised in that the handle (2) has an opening (7) in the area of the connector (6).

6. Retractor hook according to one of the claims 1 to 5, characterised in that the handle (2) has at least one finger hook (5) in the area of the extremity facing towards the hook blade (1).

7. Retractor hook according to one of the claims 1 to 6, characterised in that the handle (2) has a rim (4) at the extremity facing away from the hook blade (1).

8. Retractor hook according to one of the claims 1 to 7, characterised in that at least the extremity of the handle (2) facing towards the hook blade (1) is taperingly constructed.

9. Retractor hook according to one of the claims 1 to 8, characterised in that the hook blade (1) is constructed in one piece with the handle (2).

## Revendications

1. Ecarteur chirurgical en matériau conduisant la lumière, comprenant une lame d'écartement coudée ou en forme de crochet, possédant une face d'émission de lumière pouvant être dirigée vers l'aire de plaie, qui est structurée de manière qu'une partie au moins présente un angle inférieur à l'angle limite de la réflexion totale, et comprenant un manche relié à la lame, de même qu'une pièce de raccordement pour l'accouplement mécanique et optique de l'écarteur à un conducteur de lumière à fibre(s) ou à liquide, caractérisé en ce que le manche (2) est creux et que la cavité (9) ainsi formée présente à la fois un guide (3) pour la réception du conducteur de lumière et un raccord (6) recevant l'extrémité de sortie de lumière de ce conducteur.

2. Ecarteur selon la revendication 1, caractérisé en ce que le guide (3) est réalisé comme une saillie s'étendant dans la cavité (9) du manche (2).

3. Ecarteur selon la revendication 1 ou 2, caractérisé en ce que le guide (3) s'étend dans le sens de la longueur du manche (2).

4. Ecarteur selon une des revendications 1 à 3, caractérisé en ce que le raccord (6) destiné à recevoir l'extrémité de sortie de lumière du conducteur de lumière est de forme tubulaire.

5. Ecarteur selon une des revendications 1 à 4, caractérisé en ce que le manche (2) présente une ouverture (7) dans la région du raccord (6).

6. Ecarteur selon une des revendications 1 à 5, caractérisé en ce que le manche (2), dans la région de son extrémité tournée vers la lame d'écartement (1), présente au moins un crochet (5) pour tirer sur l'écarteur par un doigt.

7. Ecarteur selon une des revendications 1 à 6, caractérisé en ce que le manche (2) présente un bourrelet (4) à l'extrémité éloignée de la lame d'écartement (1).

8. Ecarteur selon une des revendications 1 à 7, caractérisé en ce qu'au moins l'extrémité côté lame (1) du manche (2) se rétrécit.

9. Ecarteur selon une des revendications 1 à 8, caractérisé en ce que la lame (1) est d'un seul tenant avec le manche (2).

Fig.1

Fig.2

Fig. 3